# EUROPEAN PATENT SPECIFICATION

(11) **EP 2 229 960 B1**
(45) Date of publication and mention of the grant of the patent: **08.04.2015**
(21) Application number: 08857996.6
(22) Date of filing: 04.12.2008
(51) Int. Cl.: A61K 38/10, A61K 38/18, A61L 27/22, A61L 27/54, A61L 27/60

(54) **WOUND HEALING/SKIN RECONSTRUCTION MATERIAL**
MATERIAL ZUR WUNDHEILUNG/HAUTREKONSTRUKTION
MATÉRIAU DE GUÉRISON DE BLESSURE/DE RECONSTRUCTION CUTANÉE

(30) Priority: 05.12.2007 JP 2007314640
(43) Date of publication of application: 22.09.2010
(73) Proprietor: 3-D Matrix, Ltd., Chiyoda-ku Tokyo 102-0083 (JP)
(72) Inventor: TAKAMURA, Kentaro, Tokyo 102-0083 (JP); TAKEI, Jiro, Tokyo 102-0083 (JP)
(74) Representative: Ricker, Mathias
(86) International application number: PCT/JP2008/072047
(87) International publication number: WO 2009/072556

(56) References cited:
- WO-A1-2006/116524
- WO-A1-2007/000979
- WO-A1-2008/127256
- WO-A2-02/062969
- WO-A2-2005/014615
- WO-A2-2008/073392
- JP-T- 2007 526 232
- US-B1- 6 548 630
- ANONYMOUS: 'European Journal of Dermatology' INTERNET CITATION, [Online] 31 August 1999, XP055098778 Retrieved from the Internet: <URL:http://www.jle.com/en/revues/medecine/ ejd/e-docs/00/01/86/D9/article.phtml> [retrieved on 2014-01-28]

## Description

### TECHNICAL FIELD

The invention relates to a material for use in wound healing and skin reconstruction characterized by containing a self-assembling peptide hydrogel. Also described herein is an experimental skin model using a self-assembling peptide hydrogel.

### BACKGROUND ART

For a wound treatment, recently it became known that it is effective for treating an injury to absorb exudate and protect the wound surface whilst maintaining a moist environment, and thus a wound dressing material which can protect a wound surface with maintaining a moist environment has been developed widely. For example, a film form of wound dressing material such as natural hydrophilic polymers as represented by alginic acid, water absorbent synthetic resin called hydrocolloid and porous polyurethane has been developed. Also, it has been reported that a wound dressing material comprising the hydrogel polymer having three-dimensional network structure, which consists of a polymer of water-soluble organic monomer such as N, N-dimethylacrylamide and a water swelling clay mineral, has a high ability to absorb exudate and a superior flexibility (See, JP 2007-117275).

However, the conventional wound dressing materials made of alginic acid or synthetic polymer are only for a protection of the wound surface, and almost no effect was observed on promoting the infiltration of cells involved in wound healing into the wound surface. On the other hand, a wound dressing material made of collagen used in a graft for dermal defect shows a wound healing effect by providing scaffolds for infiltration of cells relating to the wound healing to the wound surface, but there is a potential risk of infections by virus or the like because it contains an animal-derived component.

In addition, recently in the field of plastic surgery, the treatment of a skin depression, a pimple, an acne scar depression, a hypertrophic scar, a keloid, a birthmark and a nevus pigmentosus using a laser is becoming popular. Currently, in the laser treatment, right after laser irradiated, ointment is applied to the skin, the wound area is covered with an adequate dressing material, and it needs a week to reconstruct a new skin. Recently, a demand for the laser treatment is increasing, and the appearance of a skin reconstructing material which can shorten the time needed to reconstruct skin is expected.

Further, experimental animal skin models have been used in the development of pharmaceuticals and cosmetics, but their use has been restricted because of the matter of animal abuse. In 2009, the cosmetics developed by carrying out the animal test are prohibited to be sold in EU. Therefore, the development of an alternative method to the animal test is addressed worldwide in order to evaluate a pharmacological activity and safety of cosmetics. In such a situation, the development of an artificial skin model replacing the animal model is anticipated now. (See, "Biomaterials and biodevices for alternative methods to animal testing" 2007, published by CMC publishing Co., LTD)

On the other hand, recently, based on the amino acid sequence, a self-assembling peptide which can form a self-aggregate having many well-oriented peptide molecules was found, and draws attention as a new material because of its physical, chemical and biological properties. For example, it is reported that a self-assembling peptide possessing a structure, which has periodic repeats of alternating charged hydrophilic amino acids and neutral hydrophobic amino acids and thus has periodic distributions of alternating positive and negative charges, forms β-sheet structures at the low concentration of peptide solution upon exposure to physiological pH and salt concentration, and the nanofibers, about 10 nm to 20 nm in diameter, assemble to form a network structure and form a gel. This network structure has a fiber size and a pore size very similar to the natural extracellular matrix (ECM), and a use as scaffolds for cell culture has been studied. Since this peptide hydrogel is biodegradable and the degraded products have no adverse effect on the tissues, and the hydrogel has high bioabsorbability and is suitable for a cell engraftment and proliferation, and, in addition it is a synthetic chemical based product and can avoid the potential risk of animal-derived infection, it is drawing further attention as an alternatives to the collagen, as a result of the recent increase of a concern about virus transmission or other unknown infectious diseases from animals, such as bovine spongiform encephalopathy (See, teeth regeneration journal, 2005, vol.3, 1:1-11 and "Biomaterials and biodevices for alternative methods to animal testing" 2007, published by CMC publishing Co., LTD).

WO 2005/014615 A2 discloses a self-assembling peptide comprising a first amino acid domain comprising alternating hydrophobic and hydrophilic amino acids and mediating self-assembly and a second amino acid domain that does not self-assemble in isolated form.

Akimoto et al. (European Journal of Dermatology 1999, 9(5): 357-62) describe the involvement of bFGF in the pathological fibrotic process of hypertrophic scar in which fibroblasts are persistently activated.

Examples in which self-assembling peptides in combination with bFGF are applied to a skin wound for medical treatment or to skin reconstruction associated with plastic surgery have never been reported. Further, a use as an experimental skin model has never been reported either.

### DISCLOSURE OF INVENTION

An objective of the invention is to provide a bioabsorbable material for wound healing and skin reconstruction capable of healing a wound area of mammals quicker than spontaneous recovery without leaving any scars, wherein the material has no potential risk of infectious disease such as virus transmission. Another objective of the invention is to provide an experimental skin model which is an alternatives to experimental animals used in the developments of pharmaceuticals, cosmetics and treatment method in plastic surgery.

The inventor discovered that a self-assembling peptide hydrogel, which is used for the scaffolds for cell culture, has a positive effect on wound healing and skin reconstruction by applying to the wound surface, and completed the present invention.

More specifically, the invention relates to a material for use in wound healing and skin reconstruction in accordance with claim 1.

For the material for wound healing and skin reconstruction, the peptide is a self-assembling peptide having the amino acid sequence of SEQ ID NO. 1.

Also described herein is an experimental skin model of mammals containing a self-assembling peptide, wherein the peptide is an amphiphilic peptide having 8 to 200 amino acid residues with periodic repeats of alternating hydrophilic amino acids and hydrophobic amino acids, and adopts a stable β-sheet structure in an aqueous solution in the presence of a monovalent ion.

the material for wound healing and skin reconstruction and the experimental skin model further comprise biologically active agent, a basic fibroblast growth factor (bFGF)

### BRIEF DESCRIPTION OF DRAWINGS

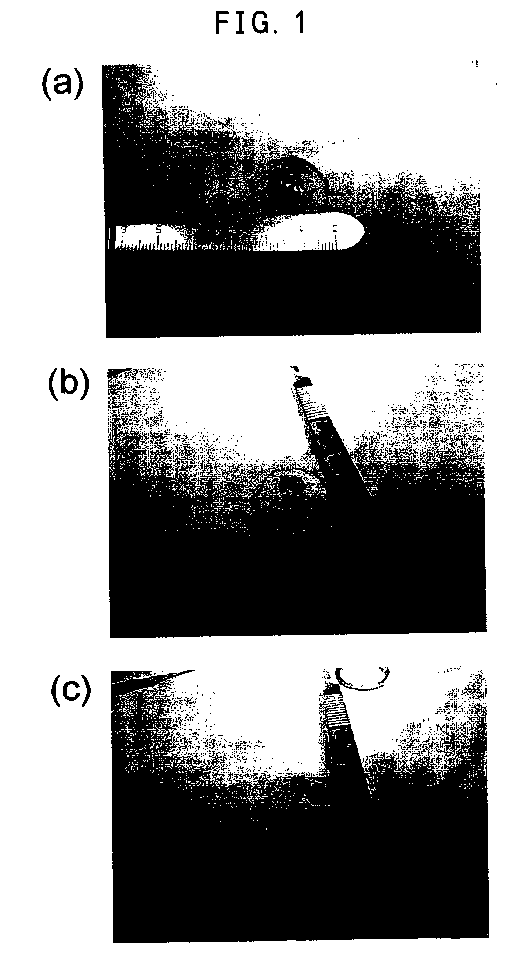

Fig. 1 is a series of photographs showing a wound area in mice on Day 0, before a treatment with a material for wound healing and skin reconstruction according to the invention. The representative images of the wound area of mice in the control group (a), the test group-1 (treated with a comparative material) (b), the test group-2 (treated with the material according to the invention) (c) are presented, and the wound of about 1 cm² where subcutaneous tissue is exposed can be observed respectively.

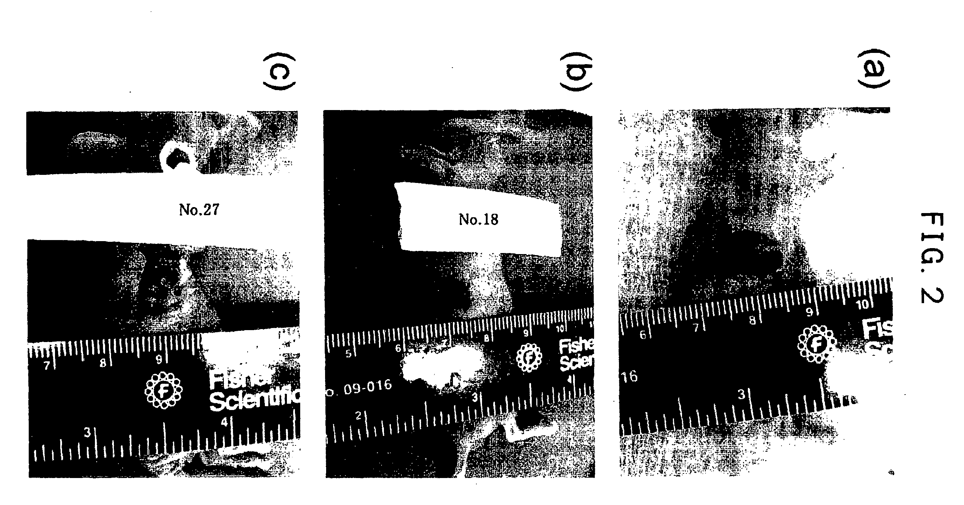

Fig. 2 is a series of photographs showing a wound area in mice on Day 7 after the treatment with the material for wound healing and skin reconstruction according to the invention. The representative images of the wound area of mice in the control group (a), the test group-1 (treated with a comparative material) (b), the test group-2 (treated with the material according to the invention) (c) are presented and correspond to the mice represented in Fig. 1. Compared to Fig. 1, a decrease of a wound area can be observed in all groups, however, it is recognized that the wound area of mice in the test group-1 (treatment with comparative material) (b) and test group-2 (c), which are treated with the material for use in wound healing and skih reconstruction, decreases drastically compared to that in the control group (a), which indicates the high healing effect.

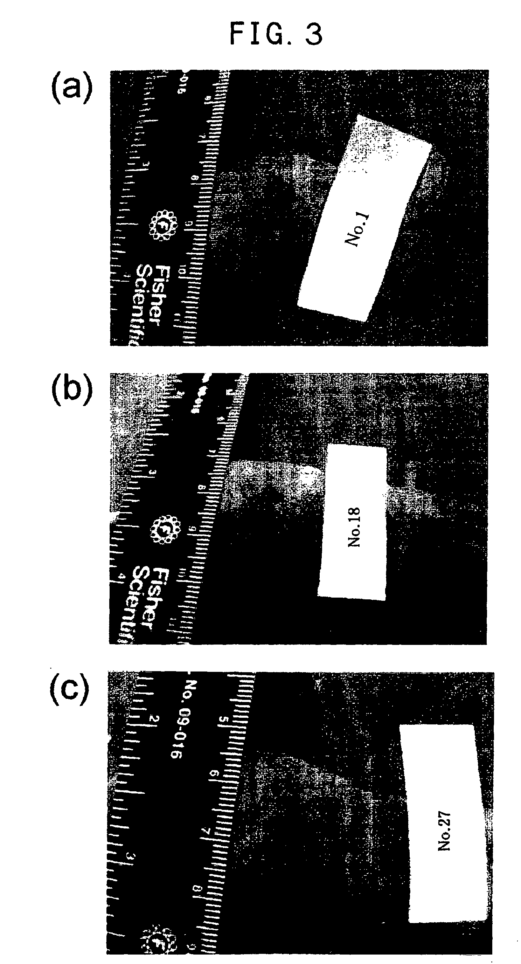

Fig. 3 is a series of photographs showing a wound area in mice on Day 14 after the treatment with the material for use in wound healing and skin reconstruction according to the invention. The representative images of the wound area of mice in the control group (a), the test group-1 (treated with a comparative material (b), the test group-2 (treated with the material according to the invention (c) are presented, and correspond to the mice represented in Fig. 1. It is recognized that the wound area of mice in the test group-1 (treatment with comparative material) (b) and test group-2 (c), which are treated with the material for use in wound healing and skin reconstruction, is almost healed. The wound area of mice in the control group (a) decreases drastically, however, a part of wound area where the epithelium formation is slightly insufficient is observed.

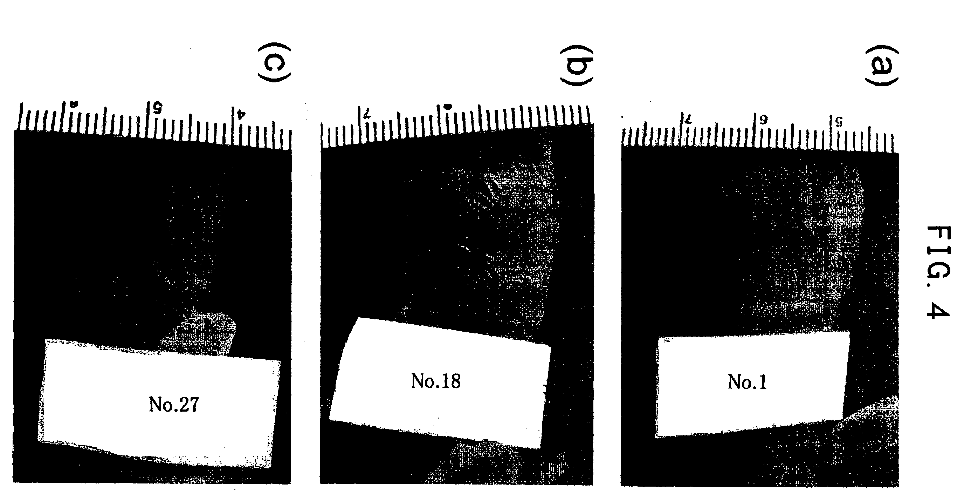

Fig. 4 is a series of photographs showing a wound area in mice on Day 21 after the treatment with the material for use in wound healing and skin reconstruction according to the invention. The representative images of the wound area of mice in the control group (a), the test group-1 (treated with a comparative material) (b), the test group-2 (treated with the material according to the invention (c) are presented and correspond to the mice represented in Fig. 1. The wounds were healed in all groups, however, it is recognized that the puckering of skin of mice in the test group-1 (treatment with comparative material) (b) and test group-2 (c), which are treated with the material for use in wound healing and skin reconstruction, is less compared to the mice in the control group.

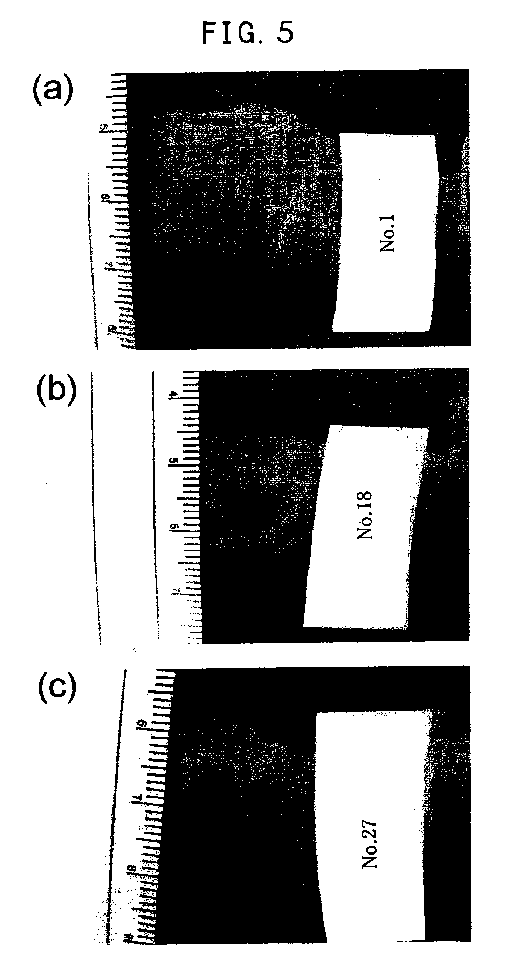

Fig. 5 is a series of photographs showing a wound area in mice on Day 28 after the treatment with the material for use in wound healing and skin reconstruction according to the invention. The representative images of the wound area of mice in the control group (a), the test group-1 (treated with a comparative material) (b), the test group-2 (treated with the material according to the invention) (c) are presented and correspond to the mice represented in Fig. 1. It is recognized that the wound area of mice in the test group-2 (c), which are treated with the material for use in wound healing and skin reconstruction, represents an ideal healing with no puckering of skin.

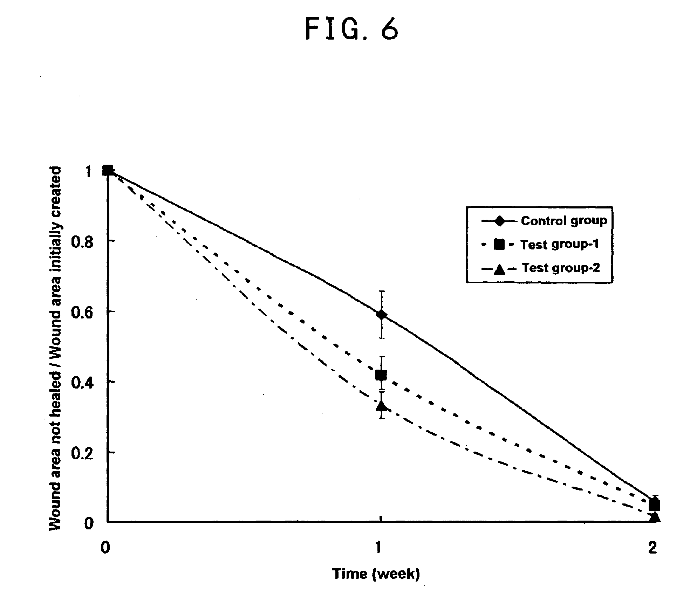

Fig. 6 is a graph representing a change of ratio of the wound area to the standard area, that is, the area when a wound created. It is recognized that the test group-1 (treaded with a comparative material) and test group-2, which is treated with the material for use in wound healing and skin reconstruction according to the invention demonstrate faster healing rate when compared to the control group.

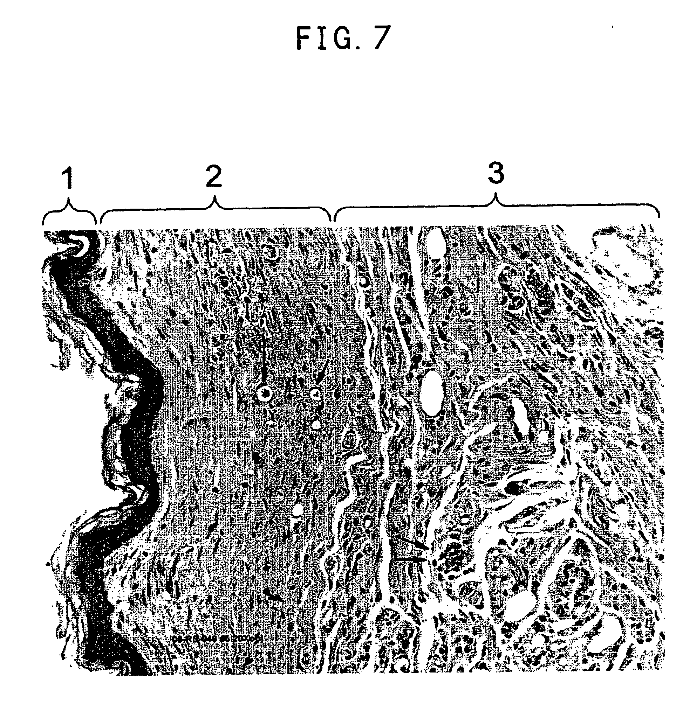

Fig. 7 is a photomicrograph of a pathological section showing a typical state of a wound area structure in the control group on Day 28 of the treatment. Capillaries are slightly prominent in the dermal layer 2 (arrows point to two examples of angiogenesis), and minimal inflammation is also observed (double arrows).

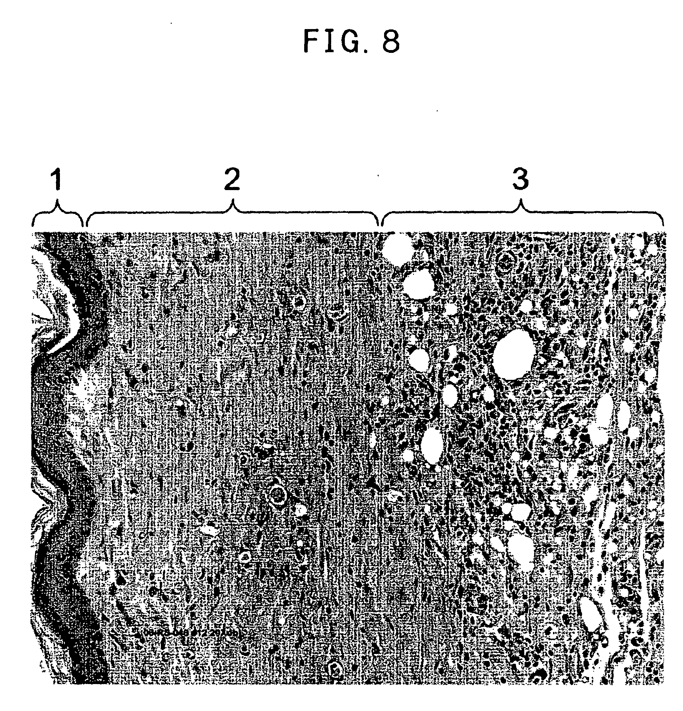

Fig. 8 is a photomicrograph of a pathological section showing a typical state of a wound area structure in the test group-1 (treatment with comparative material) on Day 28 of the treatment with a material for wound healing and skin reconstruction as described herein. Compared to the control group depicted in Fig 7, there is dense collagen so that the dermis 2 is slightly more stained.

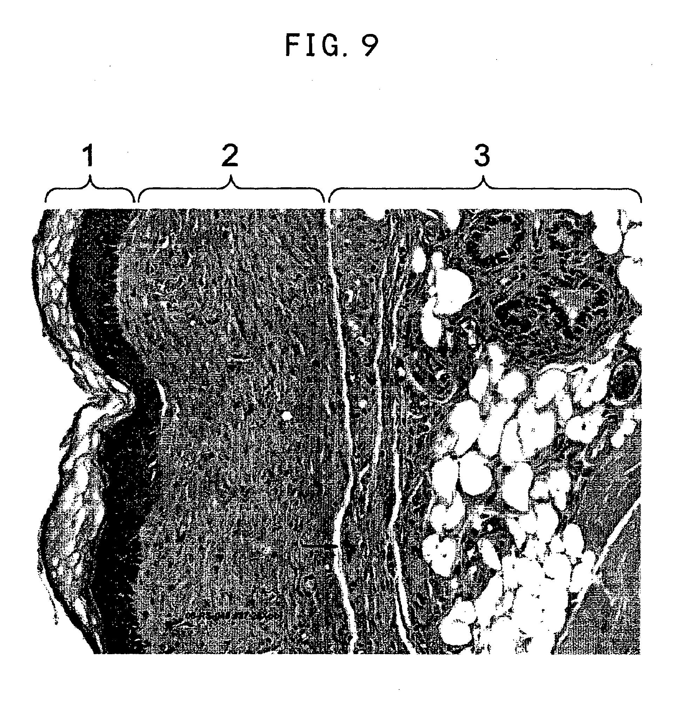

Fig. 9 is a photomicrograph of a pathological section showing a typical state of a wound area structure in the test group-2 on Day 28 of the treatment with the material for use in wound healing and skin reconstruction according to the invention. Compared to the control group depicted in Fig 7, there is dense collagen so that the dermis 2 is slightly more stained. All parameters are within normal range except for the presence of a multinucleated giant cell (arrow).

### EXPLANATION OF SYMBOLS

- 1: epidermal layer
- 2: dermal layer
- 3: subcutaneous tissue

### BEST MODE FOR CARRYING OUT THE INVENTION

A material for use in wound healing and skin reconstruction according to the present invention will be explained in detail herein.

The term "wound" used herein refers to a wound such as abrasion, burn, chap, detrition, cut, diabetic lower leg ulcer, laceration, deep cut, organ transplant, bullet wound, incision, lower leg ulcer, decubital ulcer, burn scar, scratch, burnt skin, sore skin, decubital scar, stab wound, transplant, venous ulcer or a wound associated with surgery or plastic surgery.

The term "skin reconstruction" used herein refers to a new skin construction in plastic surgery for a depression area caused by a treatment for, for example, skin depression, a pimple, an acne scar depression, a hypertrophic scar, a keloid, a birthmark and nevus pigmentosus. Further, a depression area caused by a treatment for the skin depression, a pimple, an acne scar depression, a hypertrophic scar, a keloid, a birthmark and nevus pigmentosus in plastic surgery shall here be understood to be broadly included in the definition of a wound.

A material for use in wound healing and skin reconstruction of the present invention contains a self-assembling peptide of sequence SEQ ID NO.1 and basic fibroblast growth factor as a main component,

The self-assembling peptide used in the present invention is peptide RAD16-I with the sequence of (Ac-(RADA)₄-CONH₂) (SEQ ID NO. 1) and 1 % aqueous solution of the peptide is commercially available from 3-D MATRIX, LTD. as PuraMatrix (trade mark). PuraMatrix (trade mark) contains hydrogen ion and chloride ion other than 1 % of peptide with the sequence (Ac-(RADA)₄-CONH₂) (SEQ ID NO. 1).

Since these self-assembling peptides such as PuraMatrix (trade mark) are the peptides whose peptide sequences have no specific biologically active motif, and thus there is no risk of impairing the intrinsic cell function. The biologically active motif is involved in the control of many intracellular phenomena such as transcription, and in case the biologically active motif exists, the protein in cytoplasm or on surface is phosphorylated by the enzyme that recognizes the corresponding motif. If the biologically active motif exists in a support medium, a transcription activity of various functional proteins may be inhibited. A self-assembling peptide such as PuraMatrix (trade mark), which has no biologically active motif, does not have such risks. Therefore, the self-assembling peptide such as PuraMatrix (trade mark) is useful as a material for wound healing and skin reconstruction since the peptide can serve as a matrix, which supports the various kinds of physiologically active substances secreted into a wound area, and also as scaffolds for migrating cells.

PuraMatrix (trade mark) contains an oligopeptide (Ac-(RADA)₄-CONH₂) having 16 amino acid residues with the length of about 5 nm and appears in form of liquid when pH is not higher than 5.0, but by increasing its pH above 5.0, the peptide self-assembles into nanofibers, about 10 nm in diameter, and as a result, the peptide solution forms a gel.

In a self-assembling peptide used herein, the nanofiber average diameter and pore size are 10 to 20 nm and 5 to 200 nm, on a scale very similar to the natural extracellular matrix, collagen.

PuraMatrix (trade mark) is an amphiphilic peptide having amino acid sequence with periodic repeats of alternating positively charged arginines and negatively charged aspartic acids and alternating hydrophobic alanine residues, and the spontaneous self-assembly of peptides results from hydrogen bond and hydrophobic bond between peptide molecules participated by amino acids which consist the peptides.

A condition for the spontaneous self-assembly of self-assembling peptide used herein involves physiological pH and salt concentration. Especially monovalent alkali metal ion is important. That is, a large number of sodium ion and potassium ion existing *in vivo* contributes to promote the gelation. Once gelation occurred, the gel does not decompose even under the denaturing condition for normal proteins such as elevated temperature, acid, alkaline, proteolytic enzyme or even using a denaturing agent such as urea or guanidine hydrochloride.

Since a self-assembling peptide used in the present invention is chemically synthesized, it does not contain unknown constituents attributed to animal-derived extracellular matrix. This property demonstrates that there is no potential risk of an infection including BSE and thus it also has a high safety for medical applications.

Since a self-assembling peptide consisting of natural amino acid exhibits good biocompatibility and biodegradation, it is reported that in case PuraMatrix (trade mark) is implanted into cardiac muscle of mouse, cells are embedded into injected PuraMatrix (trade mark) and normal tissue are formed. Although the degradation time depends on a condition such as injection site, fibers are degraded in 2 to 8 weeks after the injection, and excreted.

In the material for use in wound healing and skin reconstruction of the present invention, it is further added a bFGF, because it promotes the growth of fibroblast constructing skin and also it is basic itself and its biologically active function is safely sustainable in acidic self-assembling peptide solution.

Examples of states of a material for wound healing and skin reconstruction involves a solution and a gel. Since the self-assembling peptide undergoes gelation as a result of pH change of the solution, it can be distributed as a liquid agent, wherein an agent is gelated on contact with living tissue when applied. It is further possible to fix a gel on a support such as gauze or bandage and a lining, which are normally used in this technical field.

In another embodiment, a jet type spray filled up with the self-assembling peptide solution, may be produced. When such spray is sprayed to the affected area, pH of the solution increases on contact with living tissue and thus the solution undergoes gelation, it can be applied to various regions or states, comparing to handle at the state of gel.

Since a self-assembling peptide hydrogel which has a similar property as the natural extracellular matrix and can be used as scaffolds for cells showed positive skin reconstruction ability, the self-assembling peptide hydrogel can be also used as an experimental skin model.

Examples of a culture skin model include a model wherein a self-assembling peptide is gelated in multi-well culturing plate or cell culture insert for multi-well culturing plate, and epidermal tissue alone or epidermal tissue and dermal tissue together are cultivated on the gel to reconstruct the tissue structure artificially. By adding compounds or cosmetics under development into a culture skin model, the safety of the compounds and cosmetics can be assessed by observing the growth and morphology of the culturing cells.

In addition, the small molecule drugs, blood components and/or biologically active agents can be mixed into the culture skin model of the invention as needed.

A material for use in wound healing and skin reconstruction of the present invention will now be described in more detail in the following Examples, but it is understood that the following examples are not to be taken as limitations upon scope of the invention unless departing from the effect and scope of the invention

### EXAMPLES

### EXAMPLE 1

### (a) Creation of skin wound models of mice

30 NTaC:NIHS-Foxnl <nu> type nude mice (female, weight ∼20 g) were used as experimental animals. The mice were housed in the condition of a temperature of 17 to 26°C, a relative humidity of 30 to 70 %, a 12-hour light/dark cycle, minimum of ten fresh air changes/hour, and diet was provided once daily and the mice had access to water ad libitum.

Anesthesia was induced by placing the mice in a chamber filled with isoflurane at 2.5 to 4 %, and maintained with isoflurane delivered through a nose cone at 0.5 to 2.5 %.

All residual hair was removed from the upper right dorsum, and the operative area was cleaned with three alternating scrubs of povidone-iodine scrub and 70 % alcohol. Once the alternating scrubs were complete, a final application of povidone-iodine scrub was applied and allowed to dry. The area was draped for aseptic surgery.

Forceps were used to pinch up a fold of skin (at what would become the lower right corner of the wound), and scissors were used to separate the skin and create a 1 cm x 1 cm wound. Blood was soaked up with a sterile gauze pad.

### (b) Preparation of the material for use in wound healing and skin reconstruction of the invention

(b-1) The centrifuge tube was placed into a cool water bath. The bath was sonicated for 30 min, and then the contents of the tube were vortexed for one minute. Air bubbles were then removed via centrifugation (3000 rpm, 1 min). Vortexing and centrifugation were repeated as necessary to remove all bubbles. The 1 % PuraMatrix (trade mark) solution was prepared and administered to animals in test group-1.
(b-2) The 5 ng/µL hbFGF PBS solution (20 µL) was added to the sterile centrifuge tube previously containing 1 mL of 1 % PuraMatrix (trade mark). The mixture was then vortexed to homogenize. The centrifuge tube was placed into the cool water bath and sonicated for 30 min, vortexed for 1 min, then centrifuged at room temperature (3000 rpm, 1 min) to remove air bubbles. Vortexing and centrifugation were repeated as necessary to remove all air bubbles. The PuraMatrix+hbFGF solution obtained was administered to animals in the test group-2.

### (c) Treatment of wound area and observation of the affected area

The 30 skin wound model mice produced in (a) were divided into three groups of 10 mice, and treated according to the scheme in Table 1 immediately after the surgical procedure. The day when surgical procedure was performed (wound creation) was regarded as Day 0. Observation of wound area was performed at just before a treatment on Day 0 (Fig. 1), Day 7 (Fig. 2), Day 14 (Fig. 3), Day 21 (Fig. 4) and Day 28 (Fig. 5).

**TABLE 1**

| | Used reagent | Treatment |
|---|---|---|
| Control group (n=10) | Negative control | The wound area was dressed with a petroleum jelly impregnated gauze just after the wound creation on Day 0. |
| Test group-1 (n=10) | 1 % PuraMatrix (trade mark) (comparative) | 100 µL was administered to the wound area and the wound area was dressed with a petroleum jelly impregnated gauze just after the wound creation on Day 0. |
| Test group-2 (n=10) | 1 % PuraMatrix (trade mark) +hbFGF (100 ng/ 1 mL) (according to the invention) | 100 µL was administered to the wound area and the wound area was dressed with a petroleum jelly impregnated gauze just after the wound creation on Day 0. |

Baseline wound measurements were recorded prior to the application of the first dressing. Weekly measurements were obtained by removing the dressing of self-assembling hydropeptide gel (for the control group, a petroleum jelly impregnated gauze only) and exposing the wound site. A ruler was placed adjacent to the wound area and digital photographs were obtained, thereafter, the wound was re-dressed with the self-assembling hydropeptide gel (for the control group, a petroleum jelly impregnated gauze only). Wound area was calculated using ImageJ version 1.37 at NIH website (URL: http://rsb.info.nih.gov/ij/). The results are presented in Fig. 6.

Fig. 6 indicates on Day 7 and Day 14 the animals in test groups-1 and -2 were healing more quickly than those in the control group. The test group-1, that is, the treatment with PuraMatrix (trade mark) alone (comparative) had a significant healing effect visibly recognized.

On Day 7, the decrease of the wound area was observed in all groups compared to Day 0 (Fig. 1), however, the wound areas of mice in the test group-1 (treatment with comparative material) and test group-2, wherein mice were treated with a material for use in wound healing and skin reconstruction of the invention, were decreased drastically, when compared to the control group (Fig. 2).

On Day 14, healing of the wound area was almost complete in the test group-1 (treatment with comparative material) and test group-2, wherein mice were treated with a material for use in wound healing and skin reconstruction of the invention. The wound area in the control group was also decreased drastically, but a part in which the epithelial formation is slightly not enough was observed (Fig. 3). On Day 21, the wounds were completely healed in all groups, however, animals in the test group-1 (treatment with comparative material) and test group-2 (treatment with material according to the invention) show less puckering of skin when compared to the control group (Fig. 4).

Further, on Day 28 at the observation before the dissection, the scar was visible in all animals, and wound contraction was noted in all animals in the control group, 8/10 animals in the test group-1 (treatment with comparative material), and 4/10 animals in the test group-2 (treatment with material according to the invention (Fig. 5).

These results reveal that the wounds healed best in the test group-2, that is, the combination of PuraMatrix (trade mark) +hbFGF (material according to the invention), and PuraMatrix (trade mark) alone (comparative material) had a significant healing effect visibly recognized. Therefore, the combination of PuraMatrix (trade mark) +hbFGF appears to have an optimal effect on wound healing.

### (d) Histopathological observation

In order to evaluate parameters of wound healing in the skin of nude mice from three groups, histopathological examination was carried out.

After the observation on Day 28, each mouse was sacrificed and a paraffin block containing the wound area, through the center of each wound perpendicular to the apparent longest axis was produced. A hematoxylin and eosin -stained and a trichrome-stained histologic sections were prepared from each block. Using the H&E-stained section, the epithelization, vascularization, and inflammation associated with the wound were ranked, and using the trichrome-stained section the amount and quality of collagen in the area of the wound were evaluated. The results of these evaluations were shown in Table 2-1 (control group), Table 2-2 (test group-1), and Table 2-3 (test group-2).

Definitions for each score are as follows.

### <Epithelization>

0 = No re-epithelization.
1 = Partial re-epithelization. (Wound surface is not completely covered by epithelial cells.)
2 = Re-epithelization across the full surface of the wound, but the epithelium is not a stratified squamous epithelium with multiple strata (stratum basale, stratum spinosum, stratum granulosum).
3 = Re-epithelization across the full surface of the wound with a stratified squamous epithelium and keratinization, but there is evidence of active remodeling in the form of apoptotic or necrotic cells or dyskeratosis, or multiple mitotic figures, or irregular thickness or irregular strata (hyperplasia).
4 = Complete re-epithelization with a keratinized stratified squamous epithelium with remodeling and organization within normal limits.

(The only alteration is lack of hair follicles and adenexia in the dermis.)

### <Vascularization>

0 = Decreased vascularity compared to normal dermis.
1 = Dermal vascularity within normal limits.
2 = Minimally increased prominence of dermal or wound capillaries. Increased prominence may be due to increased number, dilation (congestion or hyperemia), or increased size.
3 = Mild increase in prominence of dermal or wound capillaries.
4 = Moderate increase in prominence of dermal or wound capillaries.

<Inflammation> Inflammation consisted of increased number of neutrophils and macrophages in the dermis and scar tissue beneath the epidermis. In several samples, multinucleated giant cells were observed in the wound scar, indicating a reaction to foreign material in the wound.
0 = No increase in inflammatory cells (within normal limits for dermis of nude mice).
1 = Minimal increase in inflammatory cells. Inflammation tends to be multifocal, perivascular.
2 = Mild increase in inflammatory cells. Inflammation tends to be multifocal, perivascular.
3 = Moderate increase in inflammatory cells.
4 = Marked increase in inflammatory cells.

### <Collagen quality>

0 = Lack of fibroplasia and/or collagen in wound.
1 = Minimal to mild fibroplasia in the granulation tissue of the wound.
2 = Moderate fibroplasia and collagen throughout the wound.
3 = Superficial portion of the wound has dense collagen similar to normal dermal collagen.
4 = Dense collagen throughout the wound. There are relatively fewer fibroblasts and the collagen is slightly denser than normal dermis.

### <Collagen depth>

Depth of collagen (µm) beneath the epithelium in the wound was estimated at the thickest portion of the collagen using an ocular micrometer that was calibrated with an objective micrometer (×10).

**TABLE 2-1**

| Mouse No. | Epithelization | Vascularization | Inflammation | Collagen quality | Collagen depth (µm) |
|---|---|---|---|---|---|
| 1 | 3 | 2 | 1 | 3 | 550 |
| 2 | 3 | 2 | 2 | 3 | 750 |
| 3 | 3 | 2 | 1 | 3 | 570 |
| 4 | 4 | 2 | 1 | 3 | 450 |
| 5 | 4 | 2 | 1 | 3 | 450 |
| 6 | 4 | 2 | 1 | 3 | 450 |
| 8 | 4 | 1 | 1 | 4 | 320 |
| 9 | 4 | 2 | 2 | 3 | 500 |
| 10 | 3 | 2 | 1 | 3 | 550 |

**TABLE 2-2**

| Mouse No. | Epithelization | Vascularization | Inflammation | Collagen quality | Collagen depth (µm) |
|---|---|---|---|---|---|
| 11 | 4 | 1 | 1 | 4 | 250 |
| 12 | 3 | 2 | 2 | 3 | 500 |
| 13 | 3 | 2 | 1 | 3 | 540 |
| 14 | 3 | 1 | 0 | 4 | 200 |
| 15 | 3 | 1 | 1 | 4 | 500 |
| 16 | 4 | 2 | 1 | 3 | 550 |
| 17 | 4 | 2 | 0 | 4 | 250 |
| 18 | 3 | 2 | 1 | 4 | 550 |
| 19 | 4 | 1 | 0 | 4 | 250 |
| 20 | 4 | 2 | 2 | 4 | 470 |

**TABLE 2-3**

| Mouse No. | Epithelization | Vascularization | Inflammation | Collagen quality | Collagen depth (µm) |
|---|---|---|---|---|---|
| 21 | 4 | 1 | 0 | 4 | 500 |
| 22 | 3 | 1 | 0 | 4 | 190 |
| 23 | 4 | 2 | 0 | 3 | 340 |
| 24 | 4 | 2 | 1 | 3 | 500 |
| 25 | 4 | 2 | 0 | 4 | 330 |
| 26 | 3 | 1 | 2 | 3 | 670 |
| 27 | 4 | 1 | 1 | 4 | 150 |
| 28 | 4 | 2 | 1 | 3 | 550 |
| 29 | 4 | 1 | 1 | 4 | 160 |
| 30 | 4 | 2 | 0 | 3 | 600 |

Rank scores of 4, 1, 0, and 4 for epithelization, vascularization, inflammation, and collagen quality, respectively, would indicate the most advanced wound healing. No mice in the control group and one mouse in the test group-1 (treatment with comparative material) and test group-2 (treatment with material according to the invention) had this constellation of rank scores.

There was little difference in the ranking of epithelization and vascularization across the three groups, when rank scores of three groups are compared.

As for inflammation, the larger numbers of mice are with an inflammation rank 0 in the test group-1 (treatment with comparative material) and test group-2 (treatment with material according to the invention), compared to in the control group.

The larger numbers of mice are with dense collagen throughout the wound scar in the test group-1 (treatment with comparative material) and test group-2 (treatment with material according to the invention), compared to in the control group.

The result that the test group-1 (treatment with comparative material) and test group-2 (treatment with material according to the invention) have more mice with less inflammation and with dense collagen compared to the control group may indicate these treatments promote more complete healing.

### INDUSTRIAL APPLICABILITY

The material for use in wound healing and skin reconstruction of the present invention provides higher healing promoting efficiency than simply dressing the wound surface, since the main component, self-assembling peptide, can be used as not only a dressing material but also scaffolds for migrating cells.

Further, the self-assembling peptide, the main component of a material for use in wound healing and skin reconstruction of the present invention, can be produced by synthesis, and thus there is no potential risk of infections by a virus transmission or the like in contrast to traditional aminal-derived biomaterials. Furthermore, since a self-assembling peptide itself is bioabsorbable, there is no need to concern inflammation and such.

## Claims

1. A material for use in wound healing and skin reconstruction containing a self-assembling peptide having the amino acid sequence of SEQ ID NO.1 and basic fibroblast growth factor (bFGF).

2. The material for use according to Claim 1, wherein the skin reconstruction is a new skin construction in plastic surgery for a depression area caused by a treatment for skin depression, a pimple, an acne scar depression, a hypertrophic scar, a keloid, a birthmark, and nevus pigmentosus.

## Patentansprüche

1. Material zur Verwendung bei Wundheilung und Hautrekonstruktion enthaltend ein selbstassemblierendes Peptid mit der Aminosäuresequenz der SEQ ID NO.1 und basischen Fibroblastenwachstumsfaktor (bFGF).

2. Material zur Verwendung gemäß Anspruch 1, wobei die Hautrekonstruktion eine Konstruktion neuer Haut in plastischer Chirurgie für einen Vertiefungsbereich hervorgerufen durch eine Behandlung für Hautvertiefung, einen Pickel, eine Akne-Narben-Vertiefung, eine hypertrophe Narbe, ein Keloid, ein Muttermal und Nävus pigmentosus ist.

## Revendications

1. Matériau destiné à être utilisé dans la cicatrisation d'une plaie et la reconstruction cutanée contenant un peptide auto-assemblé ayant la séquence d'acides aminés de SEQ ID NO: 1 et le facteur de croissance des fibroblastes basique (bFGF).

2. Matériau destiné à être utilisé selon la revendication 1, où la reconstruction cutanée est une construction de peau nouvelle en chirurgie plastique pour une région de dépression provoquée par le traitement d'une dépression cutanée, un bouton, une dépression due à une cicatrice d'acné, une cicatrice hypertrophique, une chéloïde, une tache de naissance, et un naevus pigmentaire.


### SEQUENCE LISTING

<110> 3-D Matrix, Ltd.
<120> Material for wound healing and skin reconstruction
<130> FP-10379PCT
<150> JP 2007-314640
   <151> 2007-12-05
<160> 1
<170> PatentIn version 3.5
<210> 1
   <211> 16
   <212> PRT
   <213> Artificial Sequence
<220>
   <223> PuraMatrix
<400> 1
   Arg Ala Asp Ala Arg Ala Asp Ala Arg Ala Asp Ala Arg Ala Asp Ala
   1 5 10 15